# EUROPEAN PATENT APPLICATION

(11) **EP 4 043 549 A2**
(43) Date of publication of application: **17.08.2022**
(21) Application number: 20846486.7
(22) Date of filing: 03.08.2020
(51) Int. Cl.: C12N 5/00, C12N 5/071

(54) **MEDIUM COMPOSITION FOR ENHANCING WNT PROTEIN ACTIVITY**

(30) Priority: 01.08.2019 KR 20190093754; 08.05.2020 KR 20200054946
(71) Applicant: Industry-Academic Cooperation Foundation, Yonsei University, Seoul 03722 (KR); Interpark Bio Convergence Corp., Seoul 08826 (KR)
(72) Inventor: LEE, Jinu, Incheon 21986 (KR); KIM, Chul Hoon, Seongnam-si Gyeonggi-do 13597 (KR); KWON, Soon Sung, Seoul 03024 (KR); YEO, Joo Hye, Seoul 06601 (KR)
(74) Representative: V.O.
(86) International application number: PCT/KR2020/010229
(87) International publication number: WO 2021/020953

(57) **Abstract**

The present disclosure relates to a serum-free medium composition for enhancing Wnt protein activity and culturing an organoid and a method for culturing an organoid using the same.

<110>    Industry-Academic Cooperation Foundation, Yonsei University

<120>    SERUM-FREE MEDIUM FOR ENHANCING WNT ACTIVITY

<130>    19PP30336

<160>    3

<170>    KoPatentIn 3.0

<210>    1
<211>    1803
<212>    DNA
<213>    Artificial Sequence

<220>
<223>    hAfamin


<400>    1
atggtgaaac tactaaaact tacaggtttt atttttttct tgtttttttt gactgaatcc        60

ctaaccctgc ccacacaacc tcgggatata gagaacttca atagtactca aaaatttata       120

gaagataata ttgaatacat caccatcatt gcatttgctc agtatgttca ggaagcaacc       180

tttgaagaaa tggaaaagct ggtgaaagac atggtagaat acaaagacag atgtatggct       240

gacaagacgc tcccagagtg ttcaaaatta cctaataatg ttttacagga aaaaatatgt       300

gctatggagg ggctgccaca aaagcataat ttctcacact gctgcagtaa ggttgatgct       360

caaagaagac tctgtttctt ctataacaag aaatctgatg tgggatttct gcctcctttc       420

cctaccctgg atcccgaaga gaaatgccag gcttatgaaa gtaacagaga atccctttta       480

aatcactttt tatatgaagt tgccagaagg aacccatttg tcttcgcccc tacacttcta       540

actgttgctg ttcattttga ggaggtggcc aaatcatgtt gtgaagaaca aaacaaagtc       600

aactgccttc aaacaagggc aatacctgtc acacaatatt taaaagcatt ttcttcttat       660

caaaaacatg tctgtggggc acttttgaaa tttggaacca aagttgtaca ctttatatat       720

attgcgatac tcagtcaaaa attccccaag attgaattta aggagcttat ttctcttgta       780

gaagatgttt cttccaacta tgatggatgc tgtgaagggg atgttgtgca gtgcatccgt       840

gacacgagca aggttatgaa ccatatttgt tcaaaacaag attctatctc cagcaaaatc       900

aaagagtgct gtgaaaagaa aataccagag cgcggccagt gcataattaa ctcaaacaaa       960

gatgatagac caaaggattt atctctaaga gaaggaaaat ttactgacag tgaaaatgtg      1020

tgtcaagaac gagatgctga cccagacacc ttctttgcga agtttacttt tgaatactca      1080

aggagacatc cagacctgtc tataccagag cttttaagaa ttgttcaaat atacaaagat      1140

ctcctgagaa attgctgcaa cacagaaaac cctccaggtt gttaccgtta cgcggaagac      1200

aaattcaatg agacaactga gaaaagcctc aagatggtac aacaagaatg taaacatttc      1260

cagaatttgg ggaaggatgg tttgaaatac cattacctca tcaggctcac gaagatagct      1320

ccccaactct ccactgaaga actggtgtct cttggcgaga aaatggtgac agctttcact 1380

acttgctgta cgctaagtga agagtttgcc tgtgttgata atttggcaga tttagttttt 1440

ggagagttat gtggagtaaa tgaaaatcga actatcaacc ctgctgtgga ccactgctgt 1500

aaaacaaact ttgccttcag aaggccctgc tttgagagtt tgaaagctga taaaacatat 1560

gtgcctccac ctttctctca agatttattt acctttcacg cagacatgtg tcaatctcag 1620

aatgaggagc ttcagaggaa gacagacagg tttcttgtca acttagtgaa gctgaagcat 1680

gaactcacag atgaagagct gcagtctttg tttacaaatt tcgcaaatgt agtggataag 1740

tgctgcaaag cagagagtcc tgaagtctgc tttaatgaag agagtccaaa aattggcaac 1800

tga 1803

<210> 2
<211> 43
<212> DNA
<213> Artificial Sequence

<220>
<223> XbaI-hAfamin U1

<400> 2
tataatctag agccaccatg gtgaaactac taaaacttac agg 43

<210> 3
<211> 32
<212> DNA
<213> Artificial Sequence

<220>
<223> hAfamin -XbaI L1

<400> 3
tatattctag attcagttgc caatttttgg ac 32

## Description

### [Technical Field]

The present disclosure relates to a medium composition for enhancing Wnt protein activity and culturing an organoid and a method for culturing an organoid using the same.

### [Background Art]

According to organoid-related technologies, theoretically, almost all types of organs can be made from stem cells only. Therefore, organoids are expected to be utilized for various diseases. Organoids may be more effective in testing the safety and efficacy of a new drug than cell tissues prepared in a two-dimensional environment. Additionally, organoids may be transplanted into damaged or underdeveloped organs and may be used to improve the condition of the subject. Accordingly, in terms of regenerative medicine, researches on organoids are on the rise recently and they are expected to be used widely in various fields.

Wnt protein, which is one of essential factors in the manufacture of organoids, generally has a lipid covalently bonded to a conserved Ser residue. Because the Wnt protein has a strongly hydrophobic property due to the lipid and tends to aggregate, it is difficult to isolate the protein in pure form. Since it was known that the Wnt protein can be obtained stably using a medium supplemented with fetal bovine serum (FBS), fetal bovine serum has been used to prepare an organoid using the Wnt protein.

However, fetal bovine serum is not suitable for long-term culturing of organoids because it contains substances that inhibit organoid growth. Therefore, the development of a medium which enables consistent growth while maintaining Wnt protein activity is necessary. In addition, since contamination by animal impurities cannot be avoided when Wnt is purified from a medium containing fetal bovine serum, the development of a medium not containing fetal bovine serum is necessary for preparation of a Wnt drug for use in human.

### [Disclosure]

### [Technical Problem]

The present disclosure is directed to providing a medium composition for culturing a cell, a cell aggregate, a tissue fragment or an organ analog, which contains a basal medium, Wnt protein or a cell expressing the same, afamin or a cell expressing the same, and albumin as active ingredients.

The present disclosure is also directed to providing a method for obtaining an organoid using the medium composition.

The present disclosure is also directed to providing a composition for enhancing Wnt protein activity in a biological sample and a composition for stabilizing Wnt protein in a biological sample.

Other purposes and advantages of the present disclosure will be more clearly understood by the following detailed description, claims and drawings.

### [Technical Solution]

In an aspect, the present disclosure relates to a medium composition for culturing a cell, a cell aggregate, a tissue fragment or an organ analog, which contains a basal medium, Wnt protein or a cell expressing the same, afamin or a cell expressing the same, and albumin as active ingredients.

In the present disclosure, the "medium (culture medium)" refers to a medium for supporting cellular growth and survival and includes all common media suitable for cell culturing.

In a specific exemplary embodiment of the present disclosure, the culture medium of the present disclosure may be a serum-free medium.

In the present disclosure, the "serum-free medium" refers to a medium which contains no serum or contains serum in less than an effective amount at which the biological and physiological functions of the serum can be exerted in the culture environment. The serum collectively refers to a serum commonly used in cell culturing such as fetal bovine serum (FBS), fetal calf serum (FCS), dialyzed fetal bovine serum, newborn calf serum (NCS), etc. The serum contains various high-molecular-weight proteins, low-molecular-weight nutrients, carriers for insoluble substances, hormones, etc., and is frequently added to common basal media. However, the medium composition of the present disclosure can maintain Wnt protein, which is essential in the culturing of organoids, etc., in active form, although it contains no serum or contains serum in less than an effective amount.

In the present disclosure, the "Wnt protein" is a cysteine-rich secreted polypeptide-based protein which affects the balance between proliferation and differentiation in many cell types and is involved in bone formation, immunoregulation, cancer, stem cell regeneration, etc. In particular, the Wnt signaling pathway is important in the regulation of tissue and organ formation in animal cells.

The Wnt protein is one of growth factors essential in the manufacture of organoids, and includes Wnt1, Wnt2, Wnt2B, Wnt3, Wnt3A, Wnt4, Wnt5A, Wnt5B, Wnt6, Wnt7A, Wnt7B, Wnt8A, Wnt8B, Wnt9A, Wnt9B, Wnt10A, Wnt10B, Wnt11 and Wnt16. Specifically, the Wnt protein may be Wnt3a protein.

In the present disclosure, the "afamin" is a glycoprotein that functions as a carrier of hydrophobic molecules in body fluids. It is an essential protein involved in the activation and dissolution of lapidated Wnt family members including Wnt1, Wnt2B, Wnt3, Wnt3A, Wnt5A, Wnt7A, Wnt7B, Wnt8, Wnt9A, Wnt9B, Wnt10A and/or Wnt10B. Besides, it may be involved in the transport of vitamin E under a condition where the lipoprotein system is not sufficient or transport of vitamin E transport across the blood-brain barrier (BBB).

A gene encoding afamin, i.e., the AFM gene, may be a gene encoding human afamin (e.g., NCBI Accession No. NP_001124) or mouse afamin (NP_660128), e.g., a gene represented by NCBI Accession No. NM_001133 or NM_145146, although not being limited thereto.

The inventors of the present disclosure have first elucidated that a combination of albumin and afamin plays a critical role in maintaining the stability and activity of Wnt protein in a culture medium. In the medium of the present disclosure, Wnt protein and afamin may be added to the medium in peptide forms or they may be provided by co-culturing cells expressing Wnt protein and/or afamin protein.

In the present disclosure the "cell expressing Wnt protein (or afamin)" encompasses not only a cell which endogenously expresses Wnt protein (or afamin) but also a transfected cell into which a nucleic acid molecule encoding the protein has been introduced by a gene delivery system.

In the present disclosure, the term "nucleic acid molecule" refers to a deoxyribonucleotide or a ribonucleotide existing as a single or double strand(s), and includes analogs of naturally occurring nucleotides unless specified otherwise (Scheit, Nucleotide Analogs, John Wiley, New York (1980); Uhlman and Peyman, Chemical Reviews, 90: 543-584 (1990)).

In the present disclosure, the term "gene delivery system" refers to a medium for expressing a desired target gene by introducing into a target cell. An ideal gene delivery system is unharmful to the human body, can be produced in large scale easily and can deliver the gene effectively.

In the present disclosure, the term "gene delivery" refers to the transport of a gene into a cell, and has the same meaning as the transduction of a gene into a cell. At the level of cells and tissues, the gene delivery has the same meaning as the spread of a gene. Accordingly, the gene delivery system of the present disclosure may also be described as a gene transduction system or a gene spread system.

When Wnt protein and afamin are provided as recombinant forms by a cell expressing the same, the two proteins may be expressed in different host cells or may be co-expressed in one host cell.

In the present disclosure, the "albumin" refers to a protein contained in serum, plasma, etc. It accounts for about 55-60% of total serum proteins. It is known that albumin maintains water content in blood by binding water. Although FBS, FCS, etc. are widely used as serum at present, safety issues have been raised because they were reported to cause human mad cow disease. In particular, when serum is used for organoid culturing, the long-term culturing of organoids is impossible because the growth of organoids is inhibited by components other than albumin and impurities. Therefore, the development of a technology for enhancing Wnt protein activity necessary for organoid culturing without using serum is necessary.

In a specific exemplary embodiment of the present disclosure, the albumin used in the present disclosure is serum albumin.

Specifically, the albumin may be contained at a concentration of 0.1-20 mg/mL. The albumin may be contained at a concentration of 0.3-20 mg/mL more specifically, and 5-20 mg/mL most specifically.

In an example of the present disclosure, it was confirmed that Wnt protein activity is increased in a concentration-dependent manner as the addition amount of albumin in a conditioned medium containing Wnt3 and a conditioned medium containing Wnt3a and afamin is increased (FIG. 1 and FIG. 2). In addition, the Wnt protein activity was increased in a concentration-dependent manner not only when bovine serum albumin was used but also when human albumin was used (FIG. 3).

This suggests that Wnt protein activity necessary for culturing of organoids can be achieved with the composition of the present disclosure although it does not contain serum such as FBS, etc. which interferes with long-term culturing of organoids.

Accordingly, the medium of the present disclosure composition may be for enhancing Wnt protein activity.

In addition, the medium of the present disclosure composition may be specifically for culturing organoids since it was confirmed that the medium of the present disclosure composition exhibits Wnt protein activity necessary for culturing of organoids although it does not contain serum.

In the present disclosure, the "organoid" refers to a cell aggregate prepared through aggregation and recombination by culturing stem cells or organ-derived cells, and may include an organoid or a cell cluster formed from a suspension cell culture. For example, it may be a stomach organoid, a small intestinal organoid, a colon organoid, a liver organoid, a thyroid organoid, a lung organoid or a brain organoid, and different organoids may be applied depending on tissue parts.

In the present disclosure, "obtainment of an organoid" includes all actions capable of producing or maintaining an organoid. For example, it may refer to survival, growth or proliferation of an organoid. It may refer to differentiation of a stem cell or a cell isolated from a specific tissue into a cell of a specific tissue or organ.

Also, in the present disclosure, the "basal medium" refers to a medium commonly known for culturing of animal cells. It may be one or more selected from a group consisting of Dulbecco's modified Eagle's medium (DMEM), minimal essential medium (MEM), basal medium Eagle (BME), RPMI 1640, F-10, F-12, DMEM-F12, α-minimal essential medium (a-MEM), Glasgow's minimal essential medium (G-MEM), Iscove's modified Dulbecco's medium (IMDM), MacCoy's 5A medium, AmnioMax, AminoMaxII complete medium, Chang's medium and MesemCult-XF medium, although not being limited thereto. If necessary, an antibiotic such as penicillin-streptomycin, a supplement, etc. may be further added.

In addition, the medium composition may further contain one or more selected from a group consisting of epidermal growth factor (EGF), noggin, thiazovivin, CHIR99021 and a pharmaceutically acceptable salt of CHIR99021, although not being limited thereto.

The CHIR99021 refers to 6-[[2-[[4-(2,4-dichlorophenyl)-5-(5-methyl-1H-imidazol-2-yl)-2-pyrimidinyl]amino]ethyl] amino]-3-pyridinecarbonitrile (CAS No. 252917-06-9), which is a compound represented by Chemical Formula 1.

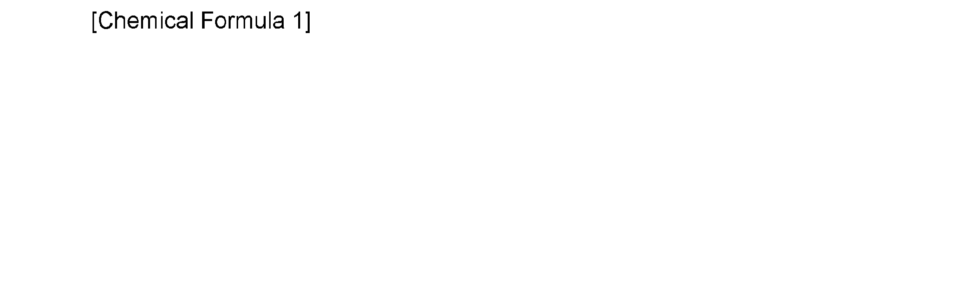

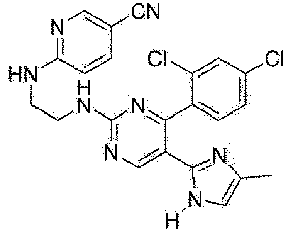

In another aspect, the present disclosure relates to a method for obtaining an organoid, which includes a step of culturing a stem cell, a population of stem cells, a cell differentiated from a stem cell or an isolated tissue fragment in the medium of the present disclosure composition.

For example, an organoid may be cultured and differentiated from multipotent stem cells or adult stem cells, and the multipotent stem cells may be embryonic stem cells or induced pluripotent stem cells.

The medium composition used for organoid culturing may further contain one or more selected from a group consisting of epidermal growth factor (EGF), noggin, thiazovivin, CHIR99021 and a pharmaceutically acceptable salt of CHIR99021, although not being limited thereto.

In another aspect, the present disclosure provides a composition for enhancing Wnt protein activity in a biological sample, which contains afamin or a cell expressing the same and albumin as active ingredients.

The description about afamin and albumin used in the present disclosure will be omitted to avoid redundancy because they were described in detail above.

In the present disclosure, the term "biological sample" refers to a sample containing Wnt protein or a cell expressing the same, obtained from a mammal including human, and includes a tissue, an organ, an organ analog, a cell or a culture thereof, although not being limited thereto.

In the present disclosure, the "enhancement of Wnt protein activity" refers to significant increase in the amount or intrinsic function of Wnt protein in the body to a detectable level as compared to a control group. The increase in activity includes not only the increase of simple function but also the increase in activity caused by increase in stability. As demonstrated in the examples described below, the composition of the present disclosure containing afamin and albumin increases and maintains not only the amount of Wnt protein in a sample but also its activity, and increases structural stability by maintaining Wnt protein in active soluble form and thereby preventing aggregation. Therefore, the term "composition for enhancing activity" used in the present disclosure may also be expressed as a "composition for enhancing stability" or a "composition for enhancing stabilizing".

### [Advantageous Effects]

Since a medium composition of the present disclosure can remarkably enhance Wnt protein activity without using serum, it can be used for long-term culturing of organoids.

It should be understood that the effect of the present disclosure is not limited to that described above but includes all effects that can be inferred from the detailed description of the present disclosure and claims.

### [Brief Description of Drawings]

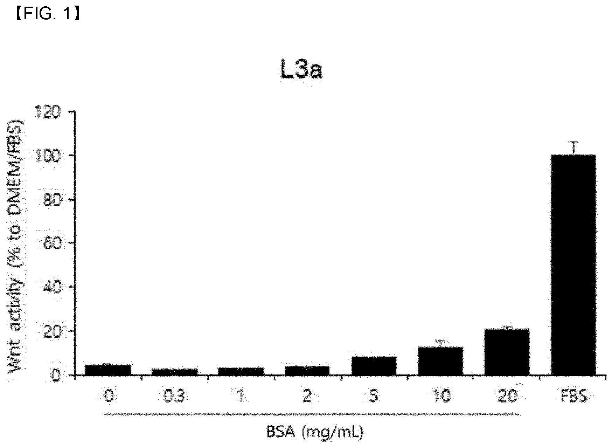

FIG. 1 shows a result of measuring the activity of Wnt protein depending on the bovine serum albumin (BSA) concentration in a conditioned medium containing Wnt3a.

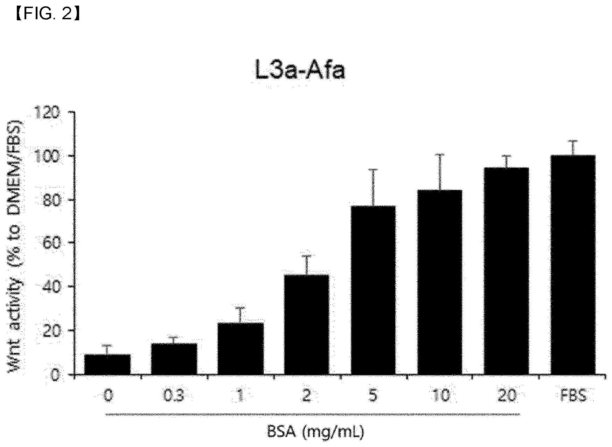

FIG. 2 shows a result of measuring the activity of Wnt protein depending on the bovine serum albumin (BSA) concentration in a conditioned medium containing Wnt3a and afamin.

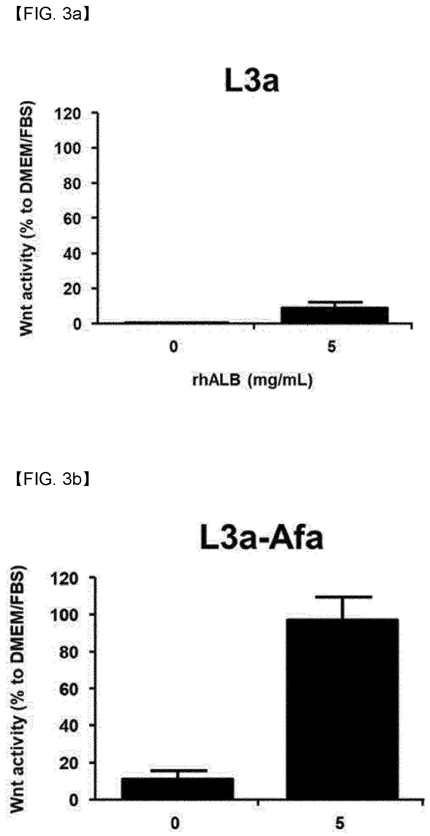

FIGS. 3a-3b show a result of measuring Wnt protein activity depending on the human albumin (rhALB) concentration in a conditioned medium. FIG. 3a shows a result for a conditioned medium containing Wnt3a, and FIG. 3b shows a result for a conditioned medium containing Wnt3a and afamin.

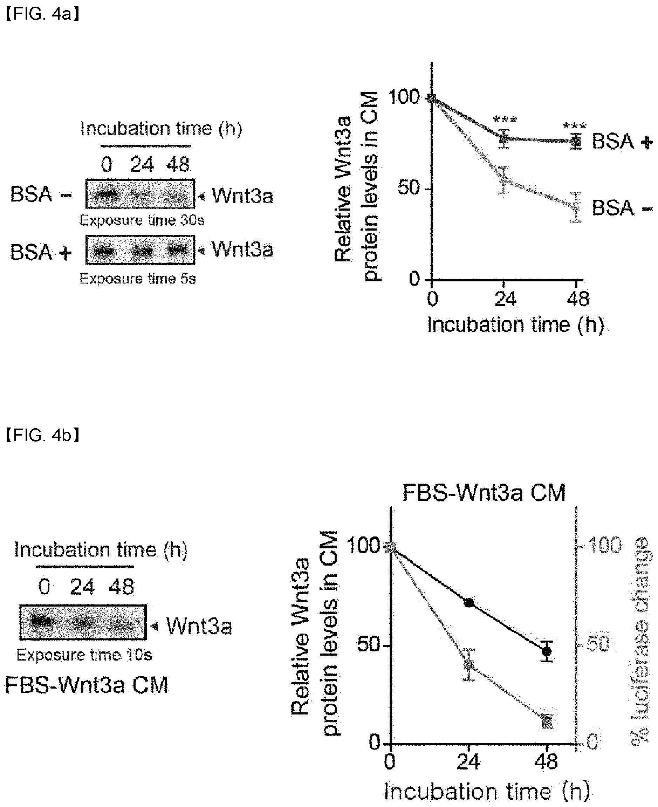

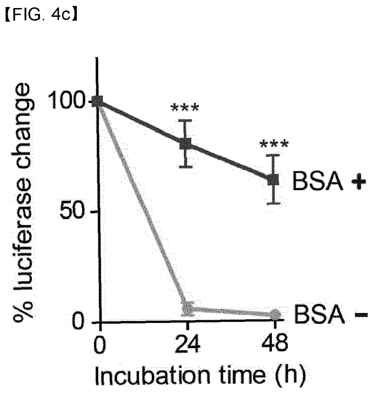

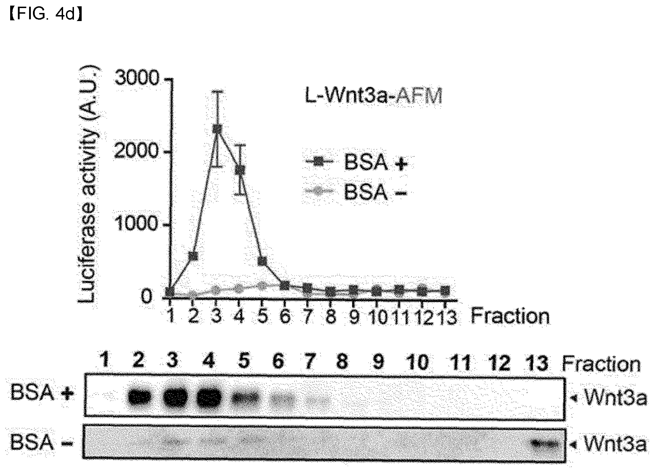

FIG. 4a and FIG. 4c show a result of culturing afamin- and Wnt3a-expresssing cells (L3a-Afa) for 5 days in the absence (-) or presence (+) of 5 mg/mL bovine serum albumin (BSA) and then measuring the stability of a conditioned medium. FIG. 4a shows a result of incubating the medium at 37 °C for 0, 24 or 48 hours and then measuring the amount of Wnt3a protein by western blot, and FIG. 4c shows a result of incubating the medium at 37 °C for 0, 24 or 48 hours and then measuring the change in the activity of Wnt protein with time. FIG. 4b shows a result of culturing cells expressing Wnt3a only (L3a) for 5 days after adding 10% fetal bovine serum (FBS) and then measuring the stability of the medium. The result of measuring the amount of Wnt3a protein by western blot after incubating the medium at 37 °C for 0, 24 or 48 hours (left, black curve) and the result of measuring the change in the activity of Wnt protein with time (right, red curve) are shown. FIG. 4d shows a result of culturing L3a-Afa cells for 5 days in the absence (-) or presence (+) of BSA and measuring the amount of Wnt3a protein in 13 fractions obtained by sucrose gradient centrifugation of the conditioned medium by western blot.

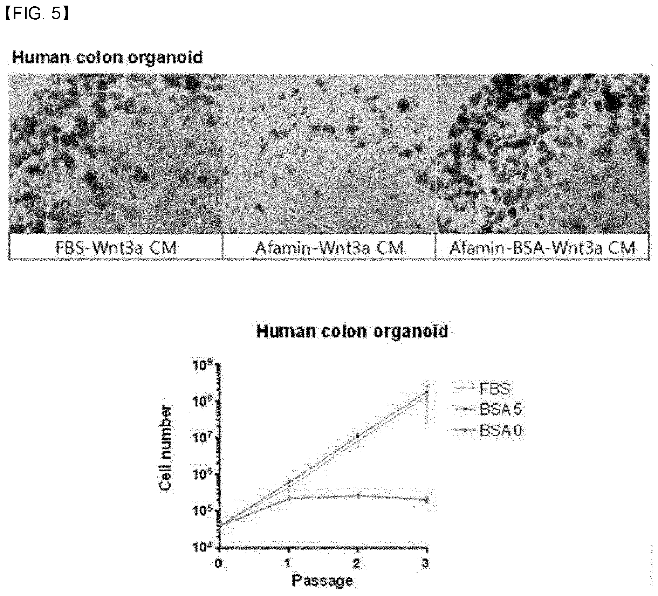

FIG. 5 shows a result of measuring the efficiency of organoid formation by culturing a human colon organoid using a conditioned medium produced by culturing L3a-Afa cells for 5 days in the absence (-) or presence (+) of BSA.

### [Best Mode]

Hereinafter, the present disclosure is described in detail through examples. However, the following examples merely illustrate the present disclosure and the present disclosure is not limited by the examples.

### Example 1. Construction of plasmid for production of human afamin (hAfamin)-expressing cells

A plasmid was constructed for production of hAfamin-expressing cells.

First, pLVX-EIBla was prepared by replacing the puromycin acetyltransferase gene of pLVX-EF1a-IRES-Puro (Clontech) with the blasticidin S deaminase gene. Then, a human afamin-coding sequence (human afamin CDS) obtained by PCR was inserted into the plasmid.

Amplification was conducted with pfu DNA polymerase (Solgent) using pCR-Bluntll-TOPO-Afamin (MHS6278-211689548, Dharmacon) as a template and SEQ ID NOS 2 and 3 as primers. After loading a sample into a PCR machine, PCR was performed under the condition of pause at 70 °C, 5 cycles of 2 minutes at 95 °C, 20 seconds at 95 °C, 20 seconds at 50 °C and 2 minutes at 68 °C, and 20 cycles of 20 seconds at 95 °C, 20 seconds at 60 °C and 2 minutes at 68 °C, followed by 3 minutes at 68 °C and pause at 4 °C. During the PCR, the sample was separated based on size on 1% agarose gel, and hAfamin was obtained through gel extraction. A hAfamin fragment was cleaved with Xba1 restriction enzyme, and then pLVX-EIBla-hAfamin plasmid was constructed by inserting into the Xba1 restriction enzyme site of the pLVXEIBla plasmid.

The hAfamin polynucleotide is represented by SEQ ID NO 1, and the primer pair used in the PCR are given in Table 1.

**[Table 1]**

| SEQ ID NO | Primer | Sequence |
|---|---|---|
| 2 | Xbal-hAfamin U1 | TCTAGA GCCACC ATGG TGAAACTACTAAAACTTACAGG |
| 3 | hAfamin -Xbal L1 | TATAT TCTAGA T TCAGTTGCCAATTTTTGGAC |

### Example 2. Construction of lentivirus for preparation of hAfamin-expressing cells

HEK293T cells were plated on a 6-well plate at a concentration of 4x105 cells/well and then transformed 24 hours later with 2 µg of DNA at a ratio of pLVX-EIBla-hAfamin : pxPAX2 (Addgene 12260) : pMD2.G (Addgene 12259) = 4 : 3 : 1. Next day, after replacing the medium and incubating for 48 hours, the culture medium was collected. The medium containing lentivirus was filtered through a 0.45-µm filter and then stored at 4 °C.

### Example 3. Preparation of afamin-expressinq cells (L-Wnt3a-Afamin, L3a-Afa)

L-Wnt3a (hereinafter, L3a) cells were acquired from Dr. Hans Clevers (Utrecht University, the Netherlands). The L3a cells are the cells designed to express Wnt3a. After culturing the L3a cells on a 24-well plate to 30% confluency, the cells were infected by mixing with 200 µL of a fresh medium and 200 µL of pLVX-EIBla-hAfamin virus. After infection for 12 hours, the medium was replaced. 72 hours later, the cells were transferred onto a 60-mm dish and treated with 10 µg/mL blasticidin for 1 week to obtain afamin-expressing cells (L3a-Afa).

### Example 4. Preparation of human albumin

Human albumin was purchased from Sigma (Catalog Number A9731).

### Example 5. Preparation of conditioned medium

A conditioned medium containing Wnt3a was obtained by culturing the L3a cells and a conditioned medium containing Wnt3a and afamin was obtained by culturing the L3a-Afa cells. Bovine serum albumin or human albumin was added to each conditioned medium at various concentrations.

### 5-1. Preparation of conditioned medium containing bovine serum albumin (BSA)

L3a cells and L3a-Afa cells were cultured respectively on a 100-mm dish until 90% confluency. After washing twice with 12 mL of PBS, the medium was replaced with a DMEM/F12 medium supplemented with 0, 0.3, 1, 2, 5, 10 or 20 mg/mL bovine serum albumin (BSA) or DMEM supplemented with 10% FBS. Then, after culturing for 5 days, the conditioned medium was collected and filtered through a 0.45-µm filter after removing the cells by centrifuging at 1,000 rpm for 3 minutes.

### 5-2. Preparation of conditioned medium containing human albumin

In consideration of the fact that impurities included in bovine serum albumin can affect Wnt protein activity, a conditioned medium was prepared using the human albumin prepared in Example 4. Specifically, L3a cells and L3a-Afa cells were cultured respectively on a 100-mm dish until 90% confluency. After washing twice with 12 mL of PBS, the medium was replaced with a DMEM/F12 medium supplemented with 0 or 5 mg/mL human albumin. Then, after culturing for 5 days, the conditioned medium was collected and filtered through a 0.45-µm filter after removing the cells by centrifuging at 1,000 rpm for 3 minutes.

### Test Example 1. Investigation of Wnt protein activity

### 1-1. Investigation of Wnt protein activity for conditioned medium containing bovine serum albumin

The Wnt protein activity of the conditioned medium prepared in Example 5-1 was measured. Specifically, reporter assay was conducted using 293-STF (ATCC) cells. 293-STF cells were plated on a 96-well plate coated with 0.05% poly-L-lysine to 70% confluency. Next day, after replacing the medium with 100 µL of a fresh medium, 50 µL of the conditioned medium was added. Luciferase assay was conducted 10-14 hours later. The measured Wnt protein activity was represented relative to the activity when fetal bovine serum (FBS) was added.

As a result, it was confirmed that, for the conditioned medium containing Wnt3, Wnt protein activity was increased as the addition amount of bovine serum albumin was increased. In particular, when bovine serum albumin was added at a concentration of 5 mg/mL or higher, Wnt protein activity was increased remarkably when the conditioned medium was added as compared to when the bovine serum albumin was not added (0 mg/mL) (FIG. 1).

In addition, it was confirmed that, for the conditioned medium containing Wnt3a and afamin, Wnt protein activity was increased remarkably as compared to the conditioned medium containing Wnt3 only. In particular, when the bovine serum albumin was further added, Wnt protein activity was increased to a level similar to that when FBS was added (FIG. 2). From this, it was confirmed that the Wnt protein activity necessary for culturing organoids can be achieved with the composition of the present disclosure which does not contain serum such as FBS, etc., which interferes with long-term culturing of organoids.

### 1-2. Investigation of Wnt protein activity for conditioned medium containing human albumin

The Wnt protein activity of the conditioned medium prepared in Example 5-2 was measured. The measurement was made according to the same method as in Test Example 1-1.

As a result, it was confirmed that Wnt3a protein activity was increased in the medium supplemented with human albumin, similarly to that supplemented with bovine serum albumin. In addition, Wnt3a protein activity was further increased in the medium supplemented with 5 mg/mL afamin and human albumin as compared to the medium not containing afamin (FIG. 3).

This result suggests that not only bovine serum albumin but also human albumin can be used, and that Wnt protein activity can be enhanced further by eliminating the effect by the impurities of bovine serum albumin.

### Test Example 2. Measurement of amount and activity of Wnt protein depending on medium composition

A conditioned medium containing Wnt3a and afamin was prepared by culturing L3a-Afa cells for 5 days in the absence (-) or presence (+) of BSA. After incubating the conditioned medium containing Wnt3a and afamin at 37 °C for 0, 24 or 48 hours, the amount of Wnt3a protein was measured by western blot using human or mouse anti-Wnt3a antibody (FIG. 4a, left). The change in the amount of Wnt3a protein depending on time was plotted (FIG. 4a, right). As a result, it was confirmed that, whereas the amount of Wnt3a was maintained in the BSA (+) medium, the amount of Wnt3a was decreased below 50% for the BSA (-) medium at 48 hours after the incubation. Through this, it can be seen that the amount of Wnt3a protein is maintained only when albumin is present in the medium (FIG. 4a).

In addition, after incubating the same conditioned medium at 37 °C for 0, 24 or 48 hours, the change in the activity of Wnt protein with time was measured by TOPflash assay (FIG. 4c). Briefly, luciferase-expressing HEK 293 STF cells, which respond to Wnt signaling, were seeded onto a 96-well plate at 5×10⁵/mL and the prepared conditioned medium was added after culturing for 24 hours. Then, Wnt activity was measured by luciferase assay 12-24 hours later. As a result, it was confirmed that, whereas the Wnt3a activity was maintained at about 70% at 48 hours for the BSA (+) medium, the activity was almost lost for the BSA (-) medium. Through this, it can be seen that a combination of afamin and albumin is essential not only in the amount of Wnt protein but also in its activity (FIG. 4c).

In addition, in order to investigate the type of Wnt protein, a conditioned medium prepared by culturing L3a-Afa cells in the absence (-) or presence (+) of BSA for 5 days was subjected to sucrose gradient centrifugation and western blot was performed on the obtained fractions. As seen from FIG. 4d, whereas the Wnt protein was inactivated in the albumin-deficient sample due to aggregation (fraction 13), the soluble functional form was maintained in the sample containing albumin (fractions 2-6), suggesting that a combination of afamin and albumin is important also in the maintenance of the active form of Wnt protein.

### Test Example 3. Measurement of human organoid formation efficiency of Wnt protein depending on medium composition

A human colon organoid was cultured by culturing L3a-Afa cells in the prepared conditioned medium for 5 days in the absence (-) or presence (+) of BSA. N-acetylcysteine (1 mM), B-27 supplement (2%), R-spondin-1 conditioned medium (10%), EGF (50 ng/mL), noggin (100 ng/mL), nicotinamide (10 mM), TGFβ inhibitor (A83-01, 500 nM) and p38 inhibitor (10 µM) were included in the conditioned medium. After placing colonic adult stem cells in a Matrigel dome, an organoid was cultured by adding the medium. The colon organoid was subcultured once in 10 days, and the efficiency of organoid formation and growth was measured by counting the number of cells. As a result, whereas the organoid formation was maintained in the BSA (+) medium, the growth of the organoid was stopped early in the BSA (-) medium. Through this, it was confirmed that the organoid is formed effectively only when the Wnt protein activity is maintained by a combination of afamin and albumin (FIG. 5).

It will be easily understood by those having ordinary knowledge in the art to which the present disclosure belongs that the foregoing description of the present disclosure is for illustration only and the present disclosure can be easily changed into other specific forms without changing the technical idea or essential features. Therefore, it should be understood that the exemplary embodiments described above are illustrative not limitative.

It is to be understood that the scope of the present disclosure is defined by the appended claims, and the meaning and scope of the claims and all changes or modifications thereof derived from equivalent concepts thereof are included in the scope of the present disclosure.

## Claims

1. A medium composition for culturing a cell, a cell aggregate, a tissue fragment or an organ analog, comprising a basal medium, Wnt protein or a cell expressing the same, afamin or a cell expressing the same, and albumin as active ingredients.

2. The medium composition according to claim 1, wherein the Wnt protein is Wnt3a protein.

3. The medium composition according to claim 1, wherein the albumin is comprised at a concentration of 0.1-20 mg/mL.

4. The medium composition according to claim 1, wherein the organ analog is an organoid.

5. The medium composition according to claim 1, wherein the medium composition is a serum-free medium composition.

6. The medium composition according to claim 1, wherein the basal medium is one or more selected from a group consisting of Dulbecco's modified Eagle's medium (DMEM), minimal essential medium (MEM), basal medium Eagle (BME), RPMI 1640, F-10, F-12, DMEM-F12, α-minimal essential medium (a-MEM), Glasgow's minimal essential medium (G-MEM), Iscove's modified Dulbecco's medium (IMDM), MacCoy's 5A medium, AmnioMax, AminoMaxII complete medium, Chang's medium and MesemCult-XF medium.

7. The medium composition according to claim 1, wherein the medium composition further comprises one or more selected from a group consisting of epidermal growth factor (EGF), noggin, thiazovivin, CHIR99021 and a pharmaceutically acceptable salt of CHIR99021.

8. A method for obtaining an organoid, comprising a step of culturing a stem cell, a population of stem cells, a cell differentiated from a stem cell, or an isolated tissue fragment in the medium composition according to any of claims 1 to 7.

9. The method for obtaining an organoid according to claim 8, wherein the medium composition further comprises one or more selected from a group consisting of epidermal growth factor (EGF), noggin, thiazovivin, CHIR99021 and a pharmaceutically acceptable salt of CHIR99021.

10. A composition for enhancing Wnt protein activity in a biological sample, comprising afamin or a cell expressing the same and albumin as active ingredients.

11. A composition for stabilizing Wnt protein in a biological sample, comprising afamin or a cell expressing the same and albumin as active ingredients.
